Europäisches Patentamt

European Patent Office

Office européen des brevets

(19)

(11) Veröffentlichungsnummer: **0 389 896**
**A2**

## (12) EUROPÄISCHE PATENTANMELDUNG

(21) Anmeldenummer: 90105046.8

(22) Anmeldetag: 17.03.90

(51) · Int. Cl.⁵: **C12M 1/34, G01N 33/48, G01N 15/14, G06F 15/62**

(30) Priorität: 22.03.89 DE 3909341

(43) Veröffentlichungstag der Anmeldung:
**03.10.90 Patentblatt 90/40**

(84) Benannte Vertragsstaaten:
**AT BE CH DE FR GB IT LI NL**

(71) Anmelder: **Schott Glaswerke**
**Hattenbergstrasse 10**
**D-6500 Mainz(DE)**

(84) **AT BE CH DE FR GB IT LI NL**

Anmelder: **Carl-Zeiss-Stiftung trading as**
**SCHOTT GLASWERKE**
**Hattenbergstrasse 10**
**D-6500 Mainz 1(DE)**

(84) **GB**

(72) Erfinder: **Langer, Peter, Dr.**
**Junkerstrasse 43**
**D-6200 Wiesbaden(DE)**
Erfinder: **Schnabel, Roland, Dr.**
**Breckenheimer Strasse 71**
**D-6238 Hofheim(DE)**
Erfinder: **Eichhorn, Dr., Uwe**
**Sertoriusring 241**
**D-6500 Mainz 21(DE)**
Erfinder: **Thielen, Andreas**
**Kaiser-Karl-Ring 34**
**D-6500 Mainz(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**D-6200 Wiesbaden(DE)**

(54) **Verfahren und Vorrichtung zur optischen Erkennung und Bewertung von Zellkolonien in Kapillaren.**

(57) In einem Verfahren zur optischen Erkennung und Bewertung von Zellkolonien in Kapillaren werden die gegenüber ihrer Umgebung unterschiedlichen Parameter der optischen Transmission längs der im Inneren mit Zellkolonien bewachsenen Kapillaren mittels computergesteuerter Bildauswertung erfaßt und auf Datenträgern gespeichert.

In einer Vorrichtung zur optischen Erkennung und Bewertung von Zellkolonien sind mehrere zur Aufnahme von Zellkolonien geeignete poröse Kapillaren in einem Modul, bestehend aus einem Perfusions- und einem Füllraum, parallel nebeneinander angeordnet. Der die Kapillaren umgebende Perfusionsraum wird von einem Nährmedium umspült, dem Substanzen zur Untersuchung ihrer Wirkung auf die Zellkolonien zugesetzt werden können.

Fig.1

## Verfahren und Vorrichtung zur optischen Erkennung und Bewertung von Zellkolonien in Kapillaren

Die vorliegende Erfindung betrifft ein Verfahren zur optischen Erkennung und Bewertung von in transparenten Körpern gezüchteten Zellkolonien mittels eines computergestützten Bildanalysesystems.

Die Erfindung betrifft auch eine Vorrichtung zur Durchführung dieses Verfahrens.

Bei der Erkennung und Bewertung von Zellen bzw. Zellkolonien wurden bisher Bildanalysesysteme verwendet, bei denen die mit Kulturen besiedelte Fläche, die sich in einer Petrischale befindet, meist nach Anfärben, durch ein Mikroskop in X- und Y-Richtung abgerastert wird. Ein solches Verfahren ist beispielsweise aus der DE-PS-3636823 bekannt. Dabei wird das Bild der Fläche durch eine Video-Kamera aufgenommen und in digitaler Form in einem Rechner gespeichert. Aus der FR-2602074-A1 ist ein ähnliches Bildanalysesystem bekannt, bei dem im Computer eine Bildauswertung durchgeführt wird, wobei zunächst interaktiv mit dem Benutzer die Bewertungskriterien festgelegt werden. Bedingt durch die große abzurasternde Fläche wird für diese Systeme ein sehr großer Speicherplatz im Computer benötigt. Wegen der zahlreichen mathematischen Operationen ist ein schneller Rechner erforderlich. Solche Verfahren sind also mit großem apparativem Aufwand sowie mit intensivem Einsatz von geschultem Personal verbunden und eignen sich daher nicht für eine routinemäßige, automatisierte Beurteilung von Zellkolonieverfahren im klinischen Labor.

Die meisten Geräte, die zur Auswertung bei der Kulturisierung auf dem Markt erhältlich sind, können universell, d.h. auch zur Bestimmung anderer Teilchen und Partikel, eingesetzt werden und sind daher sehr teuer.

Aus der US-PS 3,925,166 ist ein automatisiertes System zur Bestimmung der Sensitivität von Bakterien auf Antibiotika bekannt. Dabei werden Bakterienkolonien, die in Glaskapillaren gezüchtet werden, mit Licht von hoher Intensität durchstrahlt, wobei das von den Partikeln gestreute Licht gebündelt und von einem Fotomultiplier detektiert wird, der die elektrischen Impulse, die aus der Streuung des Lichts an den einzelnen Teilchen resultieren, zählt.

Diese Anordnung hat den Nachteil, daß nur die Kolonienanzahl mit ausreichender Sicherheit bestimmt werden kann. Eine Aussage über Größen der Kolonien ist zwar prinzipiell aufgrund der Intensität des Streulichts möglich, scheitert aber in der Praxis an der Tatsache, daß eng benachbarte Kolonien keine einzelnen Streulichtsignale, sondern ein kumuliertes Signal ergeben. Die Form von Zellkolonien ist ein jedoch wichtiges Merkmal, das mit diesem System nicht erfaßt werden kann.

Ziel der vorliegenden Erfindung ist es daher, ein Erkennungs- und Bewertungsverfahren für Zellkolonien bereitzustellen, bei dem in einfacher und kostengünstiger Weise eine routinemäßige, automatisierte Beurteilung der wesentlichen Parameter von Zellkolonien möglich ist.

Dieses Ziel wird durch das Verfahren nach Anspruch 1 und die Vorrichtung nach Anspruch 11 erreicht.

Durch das erfindungsgemäße Verfahren läßt sich eine einfache Bestimmung und Bewertung von Zellkolonien dadurch erreichen, daß mittels optischer Methoden eine örtliche und zeitliche Abhängigkeit der Kolonien so erfaßt wird, daß sowohl die Kolonienbildung als auch das Kolonienwachstum direkt sowohl quantitativ als auch qualitativ bestimmt und bewertet werden kann. Die erfaßten Daten werden in einem Rechner gespeichert und anschließend ausgewertet.

Die hier beschriebene Erfindung nutzt die Tatsache, daß sich Zellen erfolgreich in transparenten, porösen Kapillaren in Agar kultivieren lassen (vgl. DE-OS 37 33 636). Die Erkennung der Kolonien basiert auf der im Vergleich zur Umgebung unterschiedlichen optischen Transmission einer Zellkolonie, wobei der geeignete Kontrast in bekannter Weise durch Hellfeld-, Dunkelfeld- oder polarisationsoptische Betrachtung erzeugt werden kann.

Der Beleuchtungsstrahlengang kann dabei in an sich bekannter Weise gewählt werden, bspw. in einer Anordnung Lampe-Kondensor-Kapillare-Objektivlichtempfindliches Element.

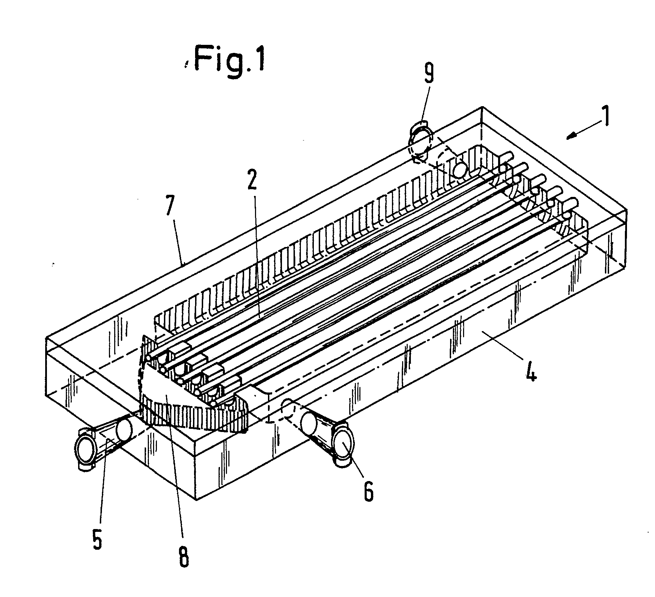

Die zur Züchtung von Kulturen verwendeten Kapillaren (Länge 3 - 20 cm, Durchmesser 0,5 - 6 mm) können einzeln vermessen werden, in einer besonders vorteilhaften Ausführungsform werden sie jedoch in einer statistisch ausreichenden Anzahl (3 - 20, bevorzugt 5 - 10) in einem Modul zusammengefaßt, in dem sie parallel nebeneinander angeordnet sind (siehe Fig. 1). Die Kapillaren bestehen aus transparentem Material, vorzugsweise aus porösem Glas. Während der Auswertung befindet sich das Modul in einem Nährmedium, bestehend aus einer wässrigen Lösung oder einem anderen Lösungsmittel (ggf. mit Zusatz einer zu untersuchenden pharmazeutischen Substanz), so daß die Poren mit dieser Flüssigkeit gefüllt sind. Dadurch sind die Brechungsindexunterschiede zwischen Glas und Poren so gering, daß die Kapillaren vollkommen transparent erscheinen. Das Modulgehäuse besteht aus lichtdurchlässigem Material und ist mit einem transparenten planen Deckel versehen, der ein Betrachten der darunter befindlichen Kapillaren durch eine spezielle Optik oder z.B. ein Mikroskop gestattet.

Die Erkennung und Bewertung der Zellkolonien geschieht nun in der Weise, daß eine Fotodiodenzeile (z.B. Firma Reticon, G-Serie) senkrecht zur Längsachse der Kapillaren außerhalb der Kapillaren selbst angeordnet wird, wobei ihre Länge so bemessen ist, daß sie alle im Modul enthaltenen Kapillaren überdeckt. Auf diese Weise ist nun nur noch ein Verschieben der Fotodiodenzeile parallel zur Kapillarlängsachse erforderlich. Diese Verschiebung geschieht vorzugsweise durch einen Schrittmotor. Nach jedem Vorschub wird die Fotodiodenzeile ausgelesen und die Intensitätswerte werden im Rechner gespeichert. Niedrige Intensität zeigt dabei an, daß sich die Diode über einer Zellkolonie befindet. Das als Intensitätsprofil vorliegende Bild der Kapillare kann nun ausgewertet werden. Durch Festlegung eines geeigneten Schwellwerts läßt sich für jede Zeile die Gesamtfläche der gesamten Kolonien folgendermaßen bestimmen :

$$F = \sum_{i=1}^{i=N} x \cdot y_i \cdot I_i$$

(F Koloniefläche pro Zeile, N Anzahl von Dioden in der Zeile, x Schrittweite des Vorschubs durch den Schrittmotor, $y_i$ Länge einer Diode). $I_i$ ist hierbei gleich 0, falls die gemessene Intensität der Diode i über dem Schwellwert und gleich 1, falls die Intensität unterhalb des Schwellwerts liegt.

Das Verfahren ist nicht auf die Anwendung einer Fotodiodenleiste beschränkt. So können bspw. auch andere lichtempfindliche Elemente verwendet werden.

Anstelle des Schrittmotors kann auch ein kontinuierlicher Vorschub über ein Federwerk, durch Gravitation oder durch einen Elektromotor erfolgen; in letzterem Fall ist ein Wegaufnehmer erforderlich, wie er z.B. üblicherweise in grafischen Zeichengeräten an Computern enthalten ist.

Durch die weiter oben beschriebene zeilenweise Verarbeitung läßt sich die Gesamtkoloniefläche ohne großen Aufwand an Speicherplatz bestimmen. Gegenüber einer über die Fläche integrierenden densitometrischen Methode, bei der in der Regel eine Anfärbung der Zellen und eine Kalibrierung der Intensität erforderlich ist, wird bei dem beschriebenen Verfahren die Koloniegrenze sicher erkannt; dies ist insbesondere bei einer kleinen Anzahl von Kolonien von Vorteil. Für eine Ermittlung des Koloniedurchmessers und der Größenverteilung können die Daten im Rechner abgelegt und anschließend mit einem Standard-Algorithmus bearbeitet werden.

Die Abbildung der Kapillaren auf der Fotodiodenzeile geschieht vorzugsweise im Maßstab 1:1 bis 1:2, da hierbei ein Arbeiten ohne Nachfokussieren möglich ist. Durch diesen kleinen Vergrößerungsmaßstab läßt sich das Problem der Schärfentiefe vernachlässigen, so daß nur einmal fokussiert werden muß. Da Zellkolonien nur im Innern der Kapillare entstehen, braucht für die Bildauswertung nur dieser Teil des Bildes berücksichtigt bzw. gespeichert zu werden. Aufgrund der bekannten und über die Länge des Moduls konstanten Abmessungen der Kapillaren kann das System erkennen, in welchem Bereich Zellkolonien zu erwarten sind. Zu diesem Zweck sind die Kapillaren am Anfang des Moduls mit einer Farbmarkierung versehen (z.B. durch Einkleben mit einer kontrastgebenden Flüssigkeit) sowie frei von Zellkolonien, so daß in diesem Bereich der Kapillardurchmesser bestimmt werden kann. Nach dem Durchfahren dieser Zone beginnen die mit Kolonien besiedelten Kapillaren.

Die Auflösung des beschriebenen Systems beträgt mit der weiter unten beschriebenen erfindungsgemäßen Diodenzeile 25x25 μm. Diese Auflösung ist ausreichend zur Bestimmung von Koloniegrößen mit einem Durchmesser von etwa 50 μm. Die Auflösung kann durch Verwendung einer Fotodiodenzeile mit kleineren Dioden oder durch eine Vergrößerung des Abbildungsmaßstabes verbessert werden. Im letzteren Fall wird allerdings die Fokussierung kritischer und kann zu Fehlbestimmungen führen.

Eine zweite Ausführungsform besteht in der Verwendung eines feststehenden zweidimensionalen Diodenarrays, wodurch die Verschiebung der Fotodiodenzeile längs der Kapillaren entfällt. Auch die Verwendung einer längeren, parallel zu den Kapillaren angeordneten Fotodiodenleiste, die senkrecht zur Kapillarachse verschoben wird, ist möglich. Dabei kann dann die Farbmarkierung zur Erkennung des Beginns des zu vermessenden Bereichs entfallen.

Die Vorrichtung zur Durchführung des erfindungsgemäßen Verfahrens besteht aus einem Modul, in dem die transparenten, porösen Kapillaren parallel nebeneinander in einer statistisch ausreichenden Anzahl angeordnet sind. Dieses Modul weist zwei getrennt voneinander angeordnete Räume, den Perfusionsraum und den Füllraum auf. Der Füllraum dient der Aufnahme der Zellsuspension, d.h., der Füllung der Kapillaren, während der Perfusionsraum der Aufnahme des Nährmediums bzw. der evtl. zuzusetzenden Substanzen dient. Das Modul weist an seinen Längsseiten im Bereich des Perfusionsraums zwei Öffnungen auf, durch die das Nährmedium zugegeben bzw. abgesaugt werden kann. Diese Zugabe bzw. das Absaugen kann sowohl stationär, d.h. in einem Schritt, als auch kontinuierlich erfolgen. Durch diese Öffnungen können der lösung gleichzeitig weitere Stoffe, wie z.B. radioaktive Marker oder Farbstoffe etc. zugegeben werden, so daß auf diese Weise die unterschiedlichsten Vitalitätsparameter der Zellkolonie erfaßbar werden.

Die Erfindung wird im folgenden anhand eines Ausführungsbeispiels in Verbindung mit den Zeichnungen näher erläutert.

Dabei zeigt

Fig. 1 das erfindungsgemäße Modul und

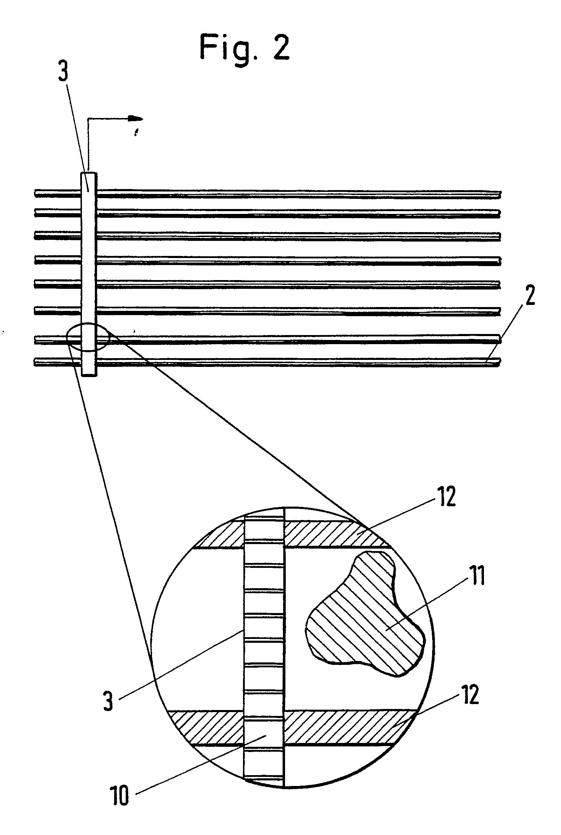

Fig. 2 eine vergrößernde Darstellung des Vorschubs der Fotodiodenleiste entlang der in dem erfindungsgemäßen Modul angeordneten Kapillaren.

Ein erfindungsgemäßes Modul (1) gemäß Fig. 1 enthält fünf poröse Glaskapillaren (2) der Länge 10 cm mit einem Innendurchmesser von 1,0 mm und einem Außendurchmesser von 1,2 mm. Der mittlere Porenradius der Kapillaren liegt bei 15 nm. Die Kapillaren (2) liegen in einem Abstand von 1,5 mm, so daß eine Breite von 12 mm mit der Diodenzeile (3) überdeckt werden muß. Die Diodenzeile (3) selbst enthält 512 Dioden und hat eine Länge von 12,8 mm, d.h., es wird ein Abbildungsmaßstab von etwa 1 hergestellt. Das Modulgehäuse (4) ist während des Auswertevorgangs mit Wasser gefüllt, die Kapillaren (2) enthalten eine wäßrige Agarlösung mit den Zellkolonien (11). Die Füllung der Kapillaren (2) geschieht in der Weise, daß die wäßrige Agarlösung durch die Einfüllöffnung (5) in den Füllraum (8) mittels einer Spritze eingebracht und anschließend unter Druck in die Kapillaren eingepreßt wird. Die Füllung des Perfusionsraums (4) geschieht durch die Einfüllöffnung (9) an der Längsseite des Modulgehäuses (7). Mit Hilfe von (nicht dargestellten), an der Einfüllöffnung (9) bzw. der Auslaßöffnung (6) angebrachten, bekannten Pump- bzw. Saugvorrichtungen kann ein kontinuierliches Umströmen der Kapillaren (2) mit frischer Lösung erreicht werden.

Zu Beginn der Auswertung wird die Diodenzeile (3) auf die Stelle der Kapillaren (2) positioniert, an der sich keine Zellkolonien befinden. Hier werden die Bereiche der Diodenzeile (3) bestimmt, auf die das Kapillarinnere abgebildet wird (relevante Bildpunkte). Die Signale der Dioden (10), auf die die Kapillarwand (12) und der Raum zwischen den Kapillaren abgebildet wird, werden zur Auswertung nicht abgespeichert.

Bei einem Innendurchmesser der Kapillaren (2) von 1 mm und einer Diodenfläche von 25 x 25 $\mu m^2$ werden also von den 512 Dioden (möglichen Bildpunkten) auf der Zeile nur etwa 200 ausgewertet.

Die Diodenzeile (3) wird nun in Schritten von 25$\mu$m über die gesamte Länge der Kapillaren (2) bewegt, wobei nach jedem Schritt die Diodenzeile mit einer Frequenz von 500 kHz ausgelesen wird und die für die Auswertng relevanten Diodensignale (Bildpunkte) abgespeichert werden. Bei einer Kapillarlänge von 10 cm sind also 4.000 Schritte notwendig.

Bei einer Abspeicherung aller relevanten Bildpunkte wird ein Datenspeicher von etwa 800 kByte benötigt. Steht dieser Speicherplatz auf dem verwendeten Rechner nicht zur Verfügung, kann das Bild ohne Schwierigkeiten zeilenweise nach jedem Schritt ausgewertet werden. Die Bestimmung der Koloniedurchmesser und der Kolonieanzahl kann mit den üblicherweise verwendeten Algorithmen realisiert werden.

## Ansprüche

1. Verfahren zur optischen Erkennung und Bewertung von in transparenten, porösen Kapillaren gezüchteten Zellkolonien, wobei die Kapillaren von einer Flüssigkeit, insbesondere Nährmedium umströmt werden, dadurch gekennzeichnet, daß mittels eines computergestützten Bildanalysesystems die gegenüber ihrer Umgebung unterschiedlichen Parameter der optischen Transmission der Zellkolonien in den Kapillaren erfaßt und auf Datenträgern gespeichert werden.

2. Verfahren nach Anspruch 1, dadurch gekennzeichnet, daß das Bildanalysesystem die Intensitätsprofile der Lichttransmission längs der Kapillaren erfaßt und speichert.

3. Verfahren nach Anspruch 1 und 2, dadurch gekennzeichnet, daß die Messung und Registrierung der Lichttransmission durch die Kapillaren bei stationärem Zustand der die Kapillaren umgebenen Flüssigkeit oder des Nährmediums erfolgen.

4. Verfahren nach Anspruch 1 und 2, dadurch gekennzeichnet, daß die Messung und Registrierung der Lichttransmission durch die Kapillaren bei Perfusion der Flüssigkeit oder des Nährmediums durch den die Kapillaren umgebenden Raum erfolgen.

5. Verfahren nach Anspruch 3 oder 4, dadurch gekennzeichnet, daß der die Kapillaren umgebenden Lösung eine Substanz zur Untersuchung ihrer Wirkung auf die Zellkolonien zugesetzt wird.

6. Verfahren nach Anspruch 5, dadurch gekennzeichnet, daß der die Kapillaren umgebenden Lösung entweder gleichzeitig oder in zeitlicher Abfolge weitere Substanzen zugesetzt werden.

7. Verfahren nach Anspruch 5 oder 6, dadurch gekennzeichnet, daß die die Kapillaren umgebenden Lösung in Bezug auf zugesetzte Substanzen einen Gradienten aufweist.

8. Verfahren nach einem der vorhergehenden Ansprüche, dadurch gekennzeichnet, daß das die Lichtparameter erfassende Element des Bildanalysesystems und die Kapillaren durch Steuermittel relativ zueinander in Richtung der Längsachse der Kapillaren bewegt werden.

9. Verfahren nach einem der Ansprüche 1-7, dadurch gekennzeichnet, daß das die Lichtparameter erfassende Element des Bildanalysesystems und die Kapillaren durch Steuermittel in Bezug auf die Längsachse der Kapillaren senkrecht zueinander bewegt werden.

10. Verfahren nach den Ansprüchen 8 oder 9, dadurch gekennzeichnet, daß das die Lichtparameter erfassende Element des Bildanalysesystems schrittweise bewegt wird.

11. Verfahren nach den Ansprüchen 8 oder 9, dadurch gekennzeichnet, daß die Bewegung des die Lichtparameter erfassende Elements des Bildanalysesystems kontinuierlich erfolgt.

12. Verfahren nach Anspruch 10, dadurch gekennzeichnet, daß die erfaßten Lichtparameter nach jedem Schritt gespeichert werden.

13. Vorrichtung zur Durchführung des Verfahrens nach einem der Ansprüche 1-12, dadurch gekennzeichnet, daß das Bildanalysesystem ein lichtempfindliches Element aufweist, welches durch einen mittels Steuermitteln gelenkten Motor über die Kapillaren bewegt wird und die durch diese transmittierenden Lichtstrahlen erfaßt und auf einem Datenträger speichert.

14. Vorrichtung nach Anspruch 13, dadurch gekennzeichnet, daß für die Beschickung von Kapillare und Kapillaraußenraum getrennt voneinander angeordnete Räume mit separaten Ein-und Auslaßöffnungen vorgesehen sind.

15. Vorrichtung nach den Ansprüchen 13 und 14, dadurch gekennzeichnet, daß mehrere Kapillaren parallel zueinander in einem Modul zusammengefaßt sind.

16. Vorrichtung nach den Ansprüchen 13-15, dadurch gekennzeichnet, daß das die Lichtparameter erfassende Element des Bildanalysesystems aus einer Fotodiodenzeile besteht.

17. Vorrichtung nach Anspruch 16, dadurch gekennzeichnet, daß die Länge der Fotodiodenzeile so bemessen ist, daß sie alle im Modul enthaltenen Kapillaren überdeckt.

18. Vorrichtung nach den Ansprüchen 13-15, dadurch gekennzeichnet, daß das die Lichtparameter erfassende Element des Bildanalysesystems eine parallel zur Kapillarlängsachse angeordnete Fotodiodenleiste ist.

19. Vorrichtung nach den Ansprüchen 13-15, dadurch gekennzeichnet, daß das die Lichtparameter erfassende Element des Bildanalysesystems ein zweidimensionales Diodenarray ist.

20. Verfahren nach den Ansprüchen 16-19, dadurch gekennzeichnet, daß die die Lichtparameter erfassenden Elemente des Bildanalysesystems die Kapillaren im Maßstab 1:1 bis 1:2 abbilden.

21. Verfahren nach den Ansprüchen 13 und 15, dadurch gekennzeichnet, daß die Länge der Kapillaren im Bereich von 3-20 cm liegt und der Durchmesser im Bereich von 0,5-6 mm.

22. Verfahren nach Anspruch 21, dadurch gekennzeichnet, daß die porösen Kapillaren aus Glas bestehen.

23. Verfahren nach den Ansprüchen 15 und 22, dadurch gekennzeichnet, daß ein Modul 3-20 Kapillaren enthält, vorzugsweise 5-10.

Fig.1

# Fig. 2